# FASCICULE DE BREVET EUROPEEN

(11) **EP 2 403 443 B1**
(45) Date de publication et mention de la délivrance du brevet: **03.09.2014**
(21) Numéro de dépôt: 10702519.9
(22) Date de dépôt: 11.01.2010
(51) Int. Cl.: A61F 9/009

(54) **ENSEMBLE POUR L'INSERTION D'UNE SONDE DANS LE CANAL LACRYMAL PAR POUSSEE DU COTE DE L'OEIL**
ANORDNUNG ZUR EINFÜHRUNG EINER SONDE IN DEN TRÄNENKANAL DURCH SCHIEBEN VON DER AUGENSEITE
ASSEMBLY FOR INSERTING A PROBE INTO THE LACRIMAL CANAL BY PUSHING FROM THE SIDE OF THE EYE

(30) Priorité: 04.02.2009 FR 0900460
(43) Date de publication de la demande: 11.01.2012
(73) Titulaire: France Chirurgie Instrumentation, 75015 Paris (FR)
(72) Inventeur: Fayet, Bruno, 75007 Paris (FR)
(74) Mandataire: Eidelsberg, Olivier Nathan
(86) Numéro de dépôt international: PCT/FR2010/000021
(87) Numéro de publication internationale: WO 2010/089472

(56) Documents cités:
- EP-A- 1 127 561
- WO-A-2007/139919
- FR-A- 2 908 042
- US-A1- 2002 151 960
- US-A1- 2004 204 704
- US-B1- 6 238 370

## Description

La présente invention se rapporte à un ensemble d'intubation monocanaliculonasal et/ou monocanaliculaire, destiné notamment à l'imperforation lacrymonasale et aux pathologies canaliculaires.

On connaît déjà dans l'art antérieur, et notamment de la demande internationale WO/2008/056060 au nom du demandeur de la présente demande, des ensembles monocanaliculaires constitués d'un mandrin en métal et d'un tube en silicone, le mandrin étant inséré dans le tube pour le pousser par le côté de l'oeil, dans le canal ou canalicule lacrymal, jusqu'à venir en contact avec la matière bouchant le canal et la traverser. Avec ce genre d'ensemble, qui permet l'insertion du tube formant sonde par le côté de l'oeil, on évite d'avoir à faire passer un mandrin métallique par le nez, ce qui peut s'avérer dangereux notamment dans le cas où l'on opère un nourrisson.

Cependant, ce genre d'ensemble à insertion par poussée présente plusieurs inconvénients. En premier lieu, en raison de l'élasticité du tube formant sonde en silicone, lorsque le mandrin y est inséré à l'intérieur et le pousse dans le canal ou canalicule lacrymal, il y a un risque d'étirement de la paroi latérale du tube en raison du frottement avec la paroi du canal. Cet étirement entraîne ensuite un retour par élasticité, donnant au tube une certaine énergie cinétique qui peut faire en sorte qu'il s'échappe du canal lacrymal dans lequel il est destiné à être disposé, ce qui peut provoquer des complications chez le patient. En deuxième lieu, il peut arriver que lors de la poussée, le mandrin vienne percer la paroi fine du tube, ce qui également peut provoquer des complications chez le patient. Un troisième inconvénient est lié au fait que le mandrin, lors de son retrait de l'intérieur du tube creux formant sonde, peut laisser passer dans le tube des germes en provenance des voies lacrymales, germes qui vont rester au sein du tube et éventuellement s'y développer, pouvant entraîner à terme également des complications. Avec un procédé sans ces inconvénients, on pourrait éviter une anesthésie générale avec ventilation mécanique assistée.

La présente invention vise à surmonter les inconvénients de l'art antérieur en proposant un ensemble d'intubation monocanaliculaire qui, bien que permettant une insertion de la sonde par le côté de l'oeil par poussée, s'avère cependant d'une utilisation plus simple pour le praticien et présentant moins de risques pour le patient.

Suivant l'invention, l'ensemble d'intubation monocanaliculonasal et/ou monocanaliculaire, destiné notamment à l'imperforation lacrymonasale, comportant :
- une sonde en un premier matériau, notamment en un matériau souple, par exemple en silicone, la sonde ayant une forme sensiblement cylindrique, par exemple cylindrique circulaire ou cylindrique elliptique, suivant un axe longitudinal, et
- des moyens d'insertion pour insérer la sonde dans un canal ou canalicule lacrymal,
est caractérisé en ce que les moyens d'insertion comportent :
- un tube d'insertion en un matériau rigide, par exemple en métal, ayant une ouverture d'extrémité distale et une ouverture d'extrémité proximale, le tube étant réalisé en forme et dimension pour pouvoir recevoir en son sein la sonde ; et
- des moyens de poussée destinés à pousser la sonde pour l'expulser du tube d'insertion par l'ouverture d'extrémité distale.

Ainsi, avec le système suivant l'invention, il n'est plus nécessaire que la sonde soit traversée et pénétrée par un mandrin métallique, ce qui permet d'éviter un percement de la paroi de la sonde. En outre, lors de la pose de la sonde, le fait qu'elle soit protégée à l'intérieur d'un tube rigide évite qu'elle soit étirée par les parois internes du canal, évitant ainsi aussi bien un déchirement qu'un déplacement en rotation ou en translation préjudiciable à son bon positionnement final. L'insertion est ainsi facilitée pour le praticien, qui n'a plus à tenir compte du frottement relatif du canal lacrymal et de la sonde en silicone qu'il souhaite y insérer, et est moins risquée pour le patient.

Suivant un mode de réalisation préféré, les moyens de poussée sont constitués d'un mandrin en un deuxième matériau, notamment plus rigide que le premier matériau, par exemple en métal, réalisé en forme et dimension pour pouvoir être inséré dans le tube d'insertion par l'ouverture proximale pour venir en contact avec la sonde insérée dans le tube, notamment sans pénétrer à l'intérieur de celle ci, de sorte que lorsque l'on fait coulisser l'un par rapport à l'autre le tube et le mandrin, la sonde est expulsée par l'ouverture de sortie distale. Suivant un autre mode de réalisation, les moyens de poussée sont constitués par un dispositif à fluide, par exemple à air comprimé, destiné à envoyer du fluide dans le tube d'insertion pour expulser la sonde.

Suivant un mode de réalisation préféré de l'invention, un bouchon de blocage est issu de la sonde, notamment sensiblement au niveau de la partie proximale de sa surface latérale longitudinale, est destiné à bloquer la sonde dans le canal lacrymal, et est constitué d'une tige à une extrémité libre de laquelle se trouve une collerette faisant saillie latéralement de la tige, et le tube d'insertion comporte sur un tronçon distal destiné à recevoir la sonde cylindrique une fente longitudinale s'étendant dans la surface latérale longitudinale du tube jusqu'à l'ouverture distale, la largeur dans la direction périphérique (transversalement à l'axe longitudinal du tube) de cette fente étant suffisante pour permettre à la tige du bouchon de blocage de coulisser le long du tube d'insertion lorsque l'on effectue un coulissement relatif mutuel du tube et du mandrin poussant la sonde cylindrique pour expulser cette dernière par l'ouverture distale.

De préférence, la longueur du tube d'insertion est sensiblement égale à deux fois la longueur de la sonde.

De préférence, le mandrin a une longueur sensiblement égale à celle du tube d'insertion.

Suivant un mode de réalisation, la sonde a sensiblement la forme d'un tube creux, dont la dimension transversale intérieure, notamment le diamètre intérieur, est inférieure à celle du mandrin de sorte que celui-ci ne peut pas pénétrer à l'intérieur du tube creux.

Suivant un autre mode de réalisation, la sonde comporte un tronçon distal en forme de tube creux, et un tronçon proximal en forme de barreau plein, dont une face arrière est destinée à être poussée par le bout distal du mandrin.

De préférence, le tube d'insertion, notamment du côté proximal comporte au moins une patte, par exemple en forme de demi disque, permettant au praticien la préhension du tube.

De préférence, le mandrin comporte, notamment sensiblement au niveau de son extrémité proximale, au moins une patte en forme d'oreille destinée à la préhension du mandrin pour permettre un déplacement relatif du mandrin dans le tube d'insertion.

Suivant un mode de réalisation préféré, la sonde sur au moins un tronçon, notamment son bulbe au niveau de son extrémité proximale, et le tube d'insertion sont cylindriques de sections transversales non circulaires sensiblement identiques, de sorte que la sonde ne peut pas subir de torsion par rapport au tube d'insertion lorsqu'elle s'y trouve, la rotation étant empêchée par les formes non circulaires des sections transversales du tube et de la sonde.

Suivant un mode de réalisation préférée, les sections transversales du tube d'insertion et d'au moins un tronçon de la sonde sont elliptiques, notamment le bulbe de la sonde disposé au niveau de son extrémité proximale.

Au dessin, donné uniquement à titre d'exemple, on décrit maintenant un mode de réalisation de l'invention.

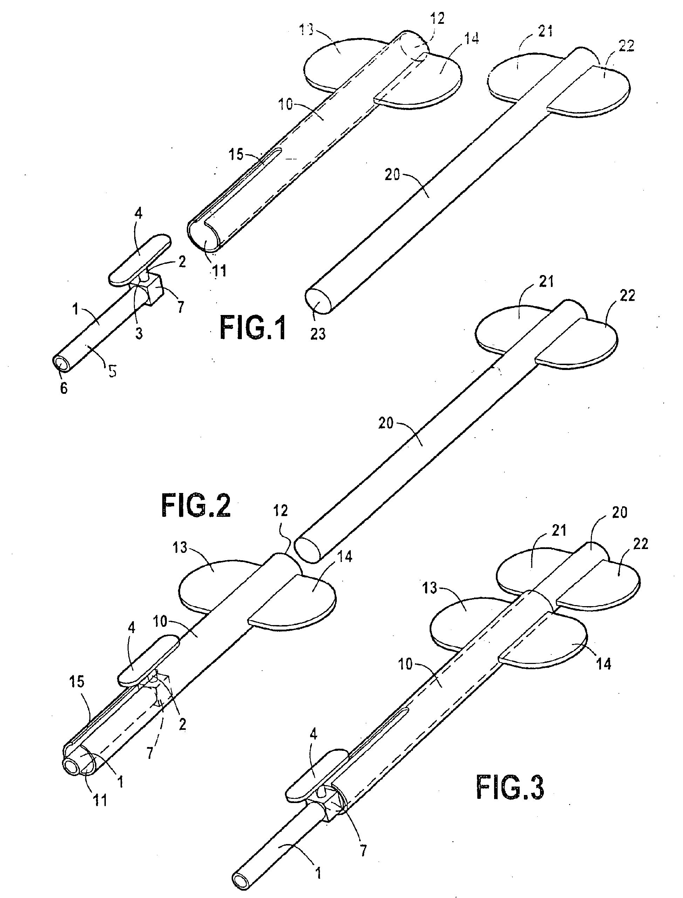

A la figure 1, il est représenté les trois constituants d'un ensemble suivant l'invention à l'état séparé des uns des autres ;

A la figure 2, il est représenté les trois constituants de la figure 1 dans un état dans lequel la sonde se trouve dans le tube d'insertion avant d'en être poussée dehors par le mandrin ; et

A la figure 3 il est représenté l'ensemble suivant l'invention pendant que la sonde est expulsée du tube d'insertion par le mandrin.

A la figure 1, il est représenté les trois constituants d'un ensemble suivant l'invention. Le premier constituant est une sonde 1 en forme de cylindre circulaire oblong, ayant un tronçon 5 distal en forme de tube creux et un tronçon 7 proximal en forme de bloc parallélépipédique, également appelé bulbe. Au niveau de l'extrémité proximale de la sonde 1, notamment au niveau du bulbe 7, fait saillie un bouchon de blocage 2 constitué d'une tige 3 s'étendant suivant un axe sensiblement transversal, notamment perpendiculaire, à l'axe longitudinal de la partie cylindrique de la sonde et d'une collerette 4 faisant saillie latéralement de la tige 3. La partie 5 cylindrique de la sonde 1 est creuse. Cependant, ceci est réalisé pour assurer une grande souplesse du tube cylindrique notamment lorsqu'il est en silicone, et on pourrait envisager, avec un matériau plus. souple, de réaliser la partie cylindrique pleine.

Le deuxième constituant est un tube 10 en matériau rigide, notamment métallique, sensiblement cylindrique circulaire ouvert à son extrémité 11 distale et à son extrémité 12 proximale. Deux pattes 13 et 14 en forme d'oreilles font saillie de la surface latérale du tube 10 d'insertion rigide au niveau de l'extrémité proximale. Sur un tronçon d'extrémité distale, s'étendant sur une longueur correspondant sensiblement à de la moitié au tiers de la longueur totale du tube d'insertion, s'étend une fente 15 qui débouche à l'ouverture 11 distale du tube d'insertion. La dimension transversale, c'est-à-dire mesurée dans la direction périphérique du tube d'insertion, de la fente 15 est telle que la tige 3 du bouchon 2 peut y passer et y coulisser lorsque la sonde 1 est poussée hors du tube d'insertion. Le diamètre intérieur du tube d'insertion est, au moins dans la partie destinée à recevoir la sonde, sensiblement égal en étant cependant supérieur, à la plus grande dimension transversale de la sonde (hors le bouchon) pour pouvoir recevoir la sonde (hors le bouchon) en son sein.

Le troisième élément constituant l'ensemble est un mandrin 20 en forme de tige, en matière métallique, de forme sensiblement cylindrique circulaire, et dont les dimensions sont telles qu'il peut être inséré à l'intérieur du tube d'insertion par l'ouverture 12 proximale et y coulisser. Le mandrin 20 comporte à son extrémité proximale deux pattes 21, 22 en forme d'oreilles destinées à sa préhension par le praticien. La dimension en longueur du mandrin est sensiblement égale à la dimension en longueur du tube rigide d'insertion.

Aux figures 2 et 3, on voit l'ensemble suivant l'invention, à l'état dans lequel la sonde 1 en silicone a été insérée dans le tronçon d'extrémité distale du tube 10 d'insertion. Dans le même temps, le mandrin 20 est inséré par l'ouverture proximale du tube 10 d'insertion de sorte que l'extrémité 23 distale du mandrin 20 vienne en contact avec la surface arrière du bloc 7 proximal de la sonde, tandis que l'extrémité 6 distale de la sonde se trouve sensiblement au niveau de l'ouverture 11 distale du tube 10 d'insertion.

Le praticien va alors insérer le tube par le côté de l'oeil, jusqu'au méat du canal ou canalicule lacrymal, pour ensuite pousser la sonde qui se trouve en son sein dans le canal lacrymal. Pour ce faire, en s'aidant des pattes 13, 14 et des pattes 21, 22, il tire vers lui le tube d'insertion tout en empêchant par poussée le mandrin tige de quitter son contact avec la sonde pour ainsi introduire celle-ci dans le canal lacrymal.

Il va en résulter un glissement ou coulissement relatif du tube d'insertion par rapport à la sonde, la tige 2 coulissant le long de la fente 15 jusqu'à expulsion total de la sonde du tube d'insertion dans le canal lacrymal.

Une fois que le chirurgien a introduit le tube à l'extrémité des voies lacrymales et a poursuivi par poussée l'avancée de la sonde dans les voies lacrymales jusqu'à ce qu'elle vienne percer l'obstruction formée dans les voies lacrymales que l'on souhaite percer pour y permettre ultérieurement le passage de larmes, l'avancée de l'ensemble est stoppé lorsque le bouchon atteint l'extrémité des voies lacrymales et que la collerette 4 vient buter contre l'épaulement ou méat lacrymal formé à l'extrémité du canal lacrymal.

On a ainsi obtenu le positionnement final de la sonde dans le canal lacrymal avec le bouchon méatique positionné au niveau de l'épaulement du méat. Le positionnement et le placement de la sonde s'est fait de manière simple par le côté de l'oeil sans passer par le nez et dans le même temps on s'assure que le mandrin ne risque pas de traverser la paroi latérale du tube sonde ni que celui-ci en raison de son étirement relatif par rapport au mandrin lors de son positionnement notamment en raison de frottement avec la paroi latérale des voies lacrymales qu'il se détache et s'échappe du canal où l'on souhaite l'insérer.

Suivant un perfectionnement, on pourrait prévoir de relier entre eux le tube d'insertion et le mandrin par un système d'articulation pouvant être commandé par poussée de sorte que le coulissement du mandrin par rapport au tube se fasse par l'intermédiaire d'une action de commande sur ce système d'articulation, par exemple par l'intermédiaire d'un bouton de poussée, voire par commande électrique ou électronique. Par exemple on pourrait utiliser un dispositif ayant un tube et des moyens de commande permettant de commander le déplacement du mandrin dans le tube. Un vissage du mandrin dans le tube et son déplacement à la manière d'une vis sans fin peut également être envisagé.

Suivant un perfectionnement, au moins une partie de la sonde, notamment le bulbe a une section transversale non circulaire et le tube d'insertion a une section sensiblement identique, également non circulaire, les dimensions relatives étant telles que la sonde est guidée par les parois du tube lors de sa poussée. La non circularité des deux sections fait que le guidage est tel que la sonde ne subit pas de rotation axiale lors de son déplacement lorsqu'elle est poussée dans le tube. La forme des deux sections du tube d'insertion et du tronçon de la sonde, notamment le bulbe, peut avantageusement être elliptique.

## Revendications

1. Ensemble d'intubation monocanaliculonasal et/ou monocanaliculaire, destiné notamment à l'imperforation lacrymonasale, comportant :
- une sonde (1) en un premier matériau, notamment en un matériau souple, par exemple en silicone, la sonde ayant une forme sensiblement cylindrique, par exemple cylindrique circulaire ou cylindrique elliptique, suivant un axe longitudinal, et
- des moyens d'insertion pour insérer la sonde dans un canal ou canalicule lacrymal,
est **caractérisé en ce que** les moyens d'insertion comportent :
- un tube (10) d'insertion en un matériau rigide, par exemple en métal, ayant une ouverture d'extrémité distale et une ouverture d'extrémité proximale, le tube étant réalisé en forme et dimension pour pouvoir recevoir en son sein la sonde ; et
- des moyens (20) de poussée destinés à pousser la sonde pour l'expulser du tube d'insertion par l'ouverture d'extrémité distale.

2. Ensemble suivant la revendication 1, **caractérisé en ce que** les moyens de poussée sont constitués d'un mandrin en un deuxième matériau, notamment plus rigide que le premier matériau, par exemple en métal, réalisé en forme et dimension pour pouvoir être inséré dans le tube d'insertion par l'ouverture proximale pour venir en contact avec la sonde insérée dans le tube de sorte que lorsque l'on fait coulisser l'un par rapport à l'autre le tube et le mandrin, la sonde est expulsée par l'ouverture de sortie distale.

3. Ensemble suivant la revendication 1 ou 2, **caractérisé en ce que** un bouchon (2) de blocage est issu de la sonde, est destiné à bloquer la sonde dans le canal lacrymal, et est constitué d'une tige (3) à une extrémité libre de laquelle se trouve une collerette (4) faisant saillie latéralement de la tige, et le tube d'insertion comporte sur un tronçon distal destiné à recevoir la sonde cylindrique une fente (15) longitudinale s'étendant dans la surface latérale longitudinale du tube jusqu'à l'ouverture distale, la largeur dans la direction périphérique (transversalement à l'axe longitudinal du tube) de cette fente étant suffisante pour permettre à la tige du bouchon de blocage de coulisser le long du tube d'insertion lorsque l'on effectue un coulissement relatif mutuel du tube et du mandrin poussant la sonde cylindrique pour expulser cette dernière par l'ouverture distale.

4. Ensemble suivant la revendication 1, 2 ou 3, **caractérisé en ce que** la sonde sur au moins un tronçon et le tube d'insertion sont cylindriques de sections transversales non circulaires sensiblement identiques, de sorte que la sonde ne peut pas subir de torsion par rapport au tube d'insertion lorsqu'elle s'y trouve, la rotation étant empêchée par les formes non circulaires des sections transversales du tube et de la sonde.

5. Ensemble suivant la revendication 2, 3 ou 4, **caractérisé en ce que** le mandrin a une longueur sensiblement égale à celle du tube d'insertion.

6. Ensemble suivant l'une des revendications 2 à 5, **caractérisé en ce que** la sonde a sensiblement la forme d'un tube creux, dont la dimension transversale intérieure, notamment le diamètre intérieur, est inférieure à celle du mandrin de sorte que celui-ci ne peut pas pénétrer à l'intérieur du tube creux.

7. Ensemble suivant l'une des revendications 1 à 6, **caractérisé en ce que** le tube d'insertion, notamment du côté proximal comporte au moins une patte, par exemple en forme d'oreille, permettant au praticien la préhension du tube.

8. Ensemble suivant l'une des revendications 2 à 7, **caractérisé en ce que** le mandrin comporte, notamment sensiblement au niveau de son extrémité proximale, au moins une patte en forme d'oreille destinée à la préhension du mandrin.

9. Ensemble suivant l'une des revendications 1 à 8, dans lequel la sonde est insérée dans un tronçon d'extrémité distale du tube d'insertion.

10. Ensemble suivant la revendication 9, **caractérisé en ce que** le tube d'insertion comporte une fente et la tige et la collerette d'un bouchon issu de la sonde font saillie à l'extérieur du tube d'insertion.

## Patentansprüche

1. Monocanaliculonasale und/oder monocanaliculare Intubationsanordnung, insbesondere zur nasolakrimalen Imperforation bestimmt, die Folgendes aufweist:
- eine Sonde (1) aus einem ersten Material, insbesondere aus einem anpassungsfähigem Material, zum Beispiel aus Silikon, wobei die Sonde entlang einer Längsachse eine im Wesentlichen zylindrische Form hat, zum Beispiel eine kreis-zylindrische oder eine ellipsen-zylindrische Form, und
- Einführmittel zum Einführen der Sonde in einen Tränenkanal oder in ein Tränenkanälchen,
**dadurch gekennzeichnet, dass** die Einführmittel Folgendes aufweisen:
- ein Einführrohr (10) aus einem starren Material, zum Beispiel aus Metall, das eine distale Endöffnung und eine proximale Endöffnung hat, wobei das Rohr in Form und Abmessung so ausgebildet ist, dass es in seinem Inneren die Sonde aufnehmen kann, und
- Schubmittel (20), die dazu bestimmt sind, die Sonde zu schieben, um sie aus dem Einführrohr durch die distale Endöffnung zu drücken.

2. Anordnung nach Anspruch 1, **dadurch gekennzeichnet, dass** die Schubmittel aus einem Dorn aus einem zweiten Material bestehen, das insbesondere starrer als das erste Material ist, zum Beispiel aus Metall, der in Form und Abmessung so ausgebildet ist, dass er durch die proximale Öffnung in das Einführrohr eingeführt werden kann, um mit der in das Rohr eingeführten Sonde in Kontakt zu kommen, dergestalt, dass beim zueinander relativen Gleiten des Rohrs und des Dorns die Sonde durch die distale Ausgangsöffnung gedrückt wird.

3. Anordnung nach Anspruch 1 oder 2, **dadurch gekennzeichnet, dass** ein Klemmstopfen (2) aus der Sonde hervortritt, der dazu bestimmt ist, die Sonde im Tränenkanal festzuklemmen, und welcher einen Stift (3) mit einem freien Ende ausbildet, an dem sich eine Auskragung (4) befindet, die seitlich des Stifts vorsteht, und, dass das Einführrohr auf einem distalen Abschnitt, der dazu bestimmt ist, die zylindrische Sonde aufzunehmen, mit einem Längsschlitz (15) versehen ist, der sich auf der Längsseitenfläche des Rohres bis zur distalen Öffnung erstreckt, wobei die Breite in der Umfangsrichtung (quer zur Längsachse des Rohres) dieses Schlitzes ausreichend ist, um das Gleiten des Stiftes des Klemmstopfens entlang dem Einführrohr zu ermöglichen, wenn man ein zueinander relatives Gleiten des Rohrs und des Dorns ausführt, bei dem die zylindrische Sonde geschoben wird, um die Letztere durch die distale Öffnung zu drücken.

4. Anordnung nach Anspruch 1, 2 oder 3, **dadurch gekennzeichnet, dass** die Sonde, auf mindestens einem Abschnitt, und das Einführrohr zylindrisch sind und im Wesentlichen identische nicht kreisrunde Querschnitte aufweisen, dergestalt, dass die Sonde keine Verdrehung in Bezug auf das Einführrohr, wenn sie sich darin befindet, erfahren kann, wobei die Rotation durch die nicht kreisrunden Formen der Querschnitte des Rohres und der Sonde verhindert ist.

5. Anordnung nach Anspruch 2, 3 oder 4, **dadurch gekennzeichnet, dass** der Dorn eine Länge hat, die im Wesentlichen mit der des Einführrohres übereinstimmt.

6. Anordnung nach einem der Ansprüche 2 bis 5, **dadurch gekennzeichnet, dass** die Sonde im Wesentlichen die Form eines hohlen Rohres hat, dessen innere Querabmessung, insbesondere der Innendurchmesser, geringer als der des Dorns ist, dergestalt, dass dieser nicht in das Innere des hohlen Rohres eindringen kann.

7. Anordnung nach einem der Ansprüche 1 bis 6, **dadurch gekennzeichnet, dass** das Einführrohr insbesondere auf der proximalen Seite mindestens eine Lasche aufweist, zum Beispiel in Form eines Flügels, wodurch dem praktizierenden Arzt das Greifen des Rohres ermöglicht wird.

8. Anordnung nach einem der Ansprüche 2 bis 7, **dadurch gekennzeichnet, dass** der Dorn, insbesondere im Wesentlichen im Bereich seines proximalen Endes, mindestens eine Lasche in Form eines Flügels aufweist, die zum Greifen des Dorns bestimmt ist.

9. Anordnung nach einem der Ansprüche 1 bis 8, bei welcher die Sonde in einen distalen Endabschnitt des Einführrohrs eingeführt ist.

10. Anordnung nach Anspruch 9, **dadurch gekennzeichnet, dass** das Einführrohr mit einem Schlitz versehen ist und der Stift und die Auskragung eines aus der Sonde hervortretenden Stopfens am Äußeren des Einführrohrs herausragen.

## Claims

1. Monocanaliculonasal and/or monocanalicular intubation assembly intended in particular for lacrimonasal imperforation comprising:
- a probe (1) made of a first material, in particular a flexible material such as silicone, the probe having a substantially cylindrical shape, for example circular cylindrical or elliptical cylindrical, along a longitudinal axis, and
- insertion means for inserting the probe into a lacrimal canal or canaliculus,
**characterised in that** the insertion means comprise:
- an insertion tube (10) made of a rigid material such as a metal, having a distal end opening and a proximal end opening, the tube having a shape and size enabling it to receive the probe therein; and
- pushing means (20) for pushing the probe and expelling said probe from the insertion tube by the distal end opening.

2. Assembly according to claim 1, **characterised in that** the pushing means consist of a mandrel made of a second material such as metal, in particular more rigid than the first material, having a shape and size enabling it to be inserted in the insertion tube by the proximal opening to make contact with the probe inserted in the tube so that when the tube and the mandrel slide relative to one another, the probe is expelled by the distal outlet opening.

3. Assembly according to claim 1 or claim 2, **characterised in that** a locking cap (2) deriving from the probe, is designed to lock the probe in the lacrimal canal and consists of a stem (3) which has at a free end a flange (4) projecting laterally from said stem, said insertion tube comprising on a distal portion for receiving the cylindrical probe a longitudinal slit (15) extending in the longitudinal lateral surface of the tube to the distal opening, the width of this slit in the peripheral direction (transversally to the longitudinal axis of the tube) being sufficient to allow the stem of the locking cap to slide along the insertion tube when the tube and the mandrel slide relative to one another, pushing the cylindrical probe and expelling it by the distal opening.

4. Assembly according to any one of claims 1, 2 or 3, **characterised in that** the probe over at least a portion and the insertion tube are cylindrical with substantially identical non-circular cross-sections, so that the probe cannot twist relative to the insertion tube when it is inside said insertion tube, rotation being prevented by the non-circular shapes of the cross-sections of the tube and the probe.

5. Assembly according to any one of claims 2, 3 or 4, **characterised in that** the mandrel has a length substantially equal to that of the insertion tube.

6. Assembly according to any one of claims 2 to 5, **characterised in that** the probe is substantially in the form of a hollow tube, of which the transverse internal dimension, in particular the internal diameter, is less than that of the mandrel so that said mandrel cannot penetrate inside the hollow tube.

7. Assembly according to any one of claims 1 to 6, **characterised in that** the insertion tube, in particular at the proximal end, comprises at least one tab, for example in the form of a lug, allowing the practitioner to grasp the tube.

8. Assembly according to any one of claims 2 to 7, **characterised in that** the mandrel, in particular substantially in the region of its proximal end, comprises at least one tab in the form of a lug for grasping the mandrel.

9. Assembly according to any one of claims 1 to 8, in which the probe is inserted in a distal end portion of the insertion tube.

10. Assembly according to claim 9, **characterised in that** the insertion tube comprises a slit and the stem and the flange of a cap derived from the probe project outside the insertion tube.
